# EUROPÄISCHE PATENTSCHRIFT

(11) **EP 3 209 232 B1**
(45) Veröffentlichungstag und Bekanntmachung des Hinweises auf die Patenterteilung: **09.10.2019**
(21) Anmeldenummer: 15794064.4
(22) Anmeldetag: 22.10.2015
(51) Int. Cl.: A61B 17/86, A61F 2/08, A61L 31/02

(54) **FIXIERUNGSIMPLANTAT**
ANCHOR IMPLANT
IMPLANT DE FIXATION

(30) Priorität: 23.10.2014 DE 102014115457
(43) Veröffentlichungstag der Anmeldung: 30.08.2017
(73) Patentinhaber: Universität Bremen, 28359 Bremen (DE)
(72) Erfinder: TUSHTEV, Kamen, 28357 Bremen (DE); SCHUMACHER, Thomas, 28205 Brermen (DE); REZWAN, Kurosch, 28357 Bremen (DE)
(74) Vertreter: Scholz, Volker
(86) Internationale Anmeldenummer: PCT/DE2015/100442
(87) Internationale Veröffentlichungsnummer: WO 2016/062307

(56) Entgegenhaltungen:
- EP-A1- 0 469 441

## Beschreibung

Die vorliegende Erfindung betrifft ein Fixierungsimplantat und Verwendungen desselben.

Es gibt eine Anzahl chirurgischer Verfahren, bei denen ein Fixierungsimplantat, wie etwa eine Schraube, ein Anker oder ein Nagel, in das Gewebe und/oder Knochen eines Patienten eingeführt wird. Dabei kann es bei verschiedenen medizinischen Eingriffen zu Situationen kommen, in denen Knochenmaterial des Patienten entfernt werden muss, um Fixierungsimplantate im Knochen zu befestigen. Ein Beispiel ist die Rekonstruktion von gerissenen Kreuzbändern, da hier das Kreuzbandtransplantat im Knochen befestigt werden muss. Dabei kann es notwendig sein, das Band entweder mit einer körpereigenen Sehne zu ersetzen oder das verletzte Band neu auszurichten und zu fixieren. Die Rekonstruktion eines Kreuzbandes im Kniegelenk ist ein bekanntes Beispiel für die Verwendung eines körpereigenen Sehnentransplantats.

Unterschiedliche Fixierungsmethoden für die Sehnentransplantate haben sich kommerziell etabliert und beinhalten sowohl gelenksnahe Varianten, wie den Einsatz von Interferenzschrauben, als auch gelenksferne Varianten, wie Varianten, die unter den Begriffen "Endobutton" oder "Transfix" bekannt sind. Insbesondere die Verwendung von Interferenzschrauben ist derzeit anerkannt und weit verbreitet. Dabei werden diese Schrauben mit Hilfe eines Schraubendrehers entlang eines zentrisch verlaufenden Führungsdrahts in einen im Knochen geschaffenen Bohrkanal eingeführt und unter ständigem Drehen bis zum Transplantat geleitet, so dass die Schraube schließlich in das Transplantat eingreift. Die so erzielte Fixierung hält das Transplantat an dem vorgesehenen Platz. Die Fixierung der Transplantate erfolgt dabei üblicherweise sowohl im Femur als auch in der Tibia.

Herkömmliche Interferenzschrauben bestehen aus Metall, wie Titan oder Edelstahl, oder Polymeren, beispielsweise Polymeren auf Basis von Milchsäure und/oder Glykolsäure.

Interferenzschrauben auf Metallbasis haben den Nachteil, dass sie aufgrund des körperfremden Materials Körperabwehrreaktionen hervorrufen können. Hierbei können Legierungselemente wie Nickel unter Umständen Autoimmunreaktionen hervorrufen. Darüber hinaus können Interferenzschrauben aus Metall aufgrund ihres scharfen selbstschneidenden Gewindes das Transplantat häufig beschädigen oder abtrennen. Auch können sich diese Interferenzschrauben im Laufe der Zeit lockern. Dieser Umstand macht eine zweite Operation zum Entfernen der Schraube und zur Behebung des entstandenen Schadens notwendig. Weiterhin kommt es aufgrund der hohen Dichteunterschiede zwischen dem Metall und dem Knochen zu Artefakten in bildgebenden Nachuntersuchungen, die eine Beurteilung des Heilungsfortganges deutlich erschweren.

Nachteilig bei polymerbasierten Interferenzschrauben ist die Freisetzung von Säuren (zum Beispiel Milchsäure) während der Resorption, die zu einem pH-bedingtem Abtöten des umliegenden Gewebes führen kann. Hierdurch entsteht eine große Kavität im Knochen, die zu einer Lockerung bzw. einem Versagen des Transplantats führt. Ein zusätzliches Problem der Fixierungsimplantate auf Polymerbasis ist die Torsionsbelastung beim Eindrehen und deren geringe Festigkeit. Hierbei kann es aufgrund der mechanischen Kräfte zum Bruch und dadurch zum Versagen des Implantats kommen. Dies führt zu einem zusätzlichen Arbeitsaufwand für den Chirurgen und einer weiteren Belastung des Patienten.

Ein Beispiel, bei dem kein körpereigenes Sehnentransplantat verwendet werden muss, um eine Bandfunktion wiederherzustellen, ist im Schulterbereich zu finden. Hierbei kommt es durch Abnutzungserscheinungen oder Überbelastung zu einer Bandverletzung oder einem Bänderriss in der Rotatorenmanschette. In diesem Fall wird das Band über mehrere im Knochen verschraubte Ausreißanker fixiert und neu ausgerichtet. Diese Ausreißanker werden bislang analog zu den oben beschriebenen Interferenzschrauben aus Metall oder Polymer hergestellt, wobei ähnliche Probleme wie bei der Kreuzbandrekonstruktion auftreten.

Aus der EP0625887 B1 ist eine Interferenzschraube bekannt, die zum Befestigen eines Dübels zur Implantatverankerung an der Verwendungsstelle in einen Tunnel einsetzbar ist, der in einen Knochen gebohrt wurde. Das auf die Schraube wirkende Drehmoment beim Einsetzen wird dadurch minimiert, dass die Schraube einen reibungsvermindernden Überzug aufweist.

Aus der EP0669110 B1 ist eine orthopädische Interferenzschraube für die Druckverankerung eines Knochen-Sehnen-Transplantats in einer Bohrung, die in einer Knochenmasse hergestellt ist, bekannt. Diese Interferenzschraube weist einen speziellen geometrischen Aufbau auf, um den Angriff der Schraube zu vergrößern und das durch die Schraube verursachte Zerfransen oder Einschneiden in Transplantate zu verringern.

Die DE102008037202 A1 beschreibt eine Interferenzschraube mit einem Außengewinde, wobei die Gewindegänge des Außengewindes zumindest abschnittsweise Ausnehmungen aufweisen.

Die EP 0 469 441 offenbart Schrauben aus keramischem Material, die zur chirurgischen Rekonstruktion von Gelenkbändern geeignet sind.

Schließlich ist aus der DE102010032808 A1 eine Zusammensetzung zur Herstellung eines Implantats bekannt, das Calciumphosphatderivat, Bindemittel und Phasenvermittler enthält. Das beschriebene Material entspricht in seiner chemischen Zusammensetzung nahezu vollständig dem menschlichen Knochenmineral. Es kann daher in den natürlichen Knochen integriert und in einer bestimmten Zeitspanne nach der Implantation durch diesen ersetzt werden. Nachteilig bei diesem Material ist jedoch dessen Sprödigkeit und geringe Festigkeit, die einen Einsatz dieses Materials als Implantat erschwert haben.

Es ist nunmehr eine Aufgabe der vorliegenden Erfindung, ein Fixierungsimplantat bereitzustellen, das die Nachteile des Stands der Technik überwindet und insbesondere überwiegend aus einem keramischen Material aufgebaut ist und zufriedenstellende mechanische Eigenschaften besitzt. Ferner soll das Fixierungsimplantat ohne große Torsionsbelastung in den Knochen eingeführt werden können.

Diese Aufgabe wird gelöst durch ein Fixierungsimplantat mit einem Implantatkörper, der im Wesentlichen aus keramischem Material aufgebaut ist und ein Drallaußengewinde aufweist, das mehr als einen Gewindesteg aufweist und wobei ein Gewindesteigungswinkel des Drallaußengewindes im Bereich von 30° bis 90° ist.

Unter einem Drallaußengewinde ist im Rahmen der vorliegenden Erfindung ein Schraubengewinde zu verstehen, das auf seiner Außenseite mindestens zwei, im wesentlichen parallele Gewindestege aufweist, die um die Implantatachse tordiert sind, sich im wesentlichen von einem Schraubenkopf zu einer Schraubenspitze spiralförmig erstrecken, mit einem Gewindesteigungswinkel von 30° bis 90°.

Erfindungsgemäß ist dabei bevorzugt, dass das Fixierungsimplantat eine Interferenzschraube oder ein Anker, wie ein Fadenanker, ist. Bei einer Interferenzschraube oder einem Fadenanker handelt es sich um eine Fixierungsform, welche in eine zuvor gesetzte Bohrung eingedreht wird und nach dem Eindrehen durch eine Übermaßpassung in der Bohrung fixiert ist. Entsprechende Interferenzschrauben und Fadenanker sind auf dem Fachgebiet hinlänglich bekannt.

Bevorzugt ist, dass das keramische Material ein biokompatibles keramisches Material, vorzugsweise ein Material auf Basis von Calciumphosphaten, Calciumsilikaten, Magnesiumsilikaten, Zinksilikaten und/oder Strontiumsilikaten, ist.

Der Begriff "biokompatibel", wie er hierin verwendet wird, bezeichnet ein keramisches Material, das beispielsweise bioaktiv oder bioinert ist. Als biokompatibel werden Materialien bezeichnet, die keinen negativen Einfluss auf menschliches Zellgewebe haben. Eine "Bioaktivität" drückt die Fähigkeit des keramischen Materials aus, eine direkte Anbindung an das menschliche Gewebe zu ermöglichen.

Besonders bevorzugt kann das keramische Material auf Basis von Calciumphosphaten ausgewählt sein aus der Gruppe bestehend aus Hydroxylapatit, Tricalciumphosphat, Carbonatapatit, Fluorapatit und/oder Kombinationen derselben.

Bevorzugt ist das keramische Material ein Komposit-Material auf Basis von Polymer und keramischem Material oder Metall und keramischem Material.

In einer bevorzugten Ausführungsform weist das Drallaußengewinde 2, 3, 4, 5 oder mehr im Wesentlichen parallel zueinander angeordnete Gewindestege auf.

Bevorzugt weist der Implantatkörper einen axialen zentrischen Kanal auf.

Ebenfalls bevorzugt ist, dass der Implantatkörper im Wesentlichen konisch oder zylinderisch ausgebildet ist.

Besonders bevorzugt ist, dass das Drallaußengewinde sich im Wesentlichen über die gesamte Länge des Implantatkörpers erstreckt.

Besonders bevorzugt ist, dass der Gewindesteigungswinkel des Drallaußengewindes im Bereich von mindestens 45°, vorzugsweise 60° bis 90° liegt.

Besonders bevorzugt ist das Einbringen des Fixierungsimplantats über eine Druckbelastung, die ohne Drehwerkzeug aufgebracht werden kann.

Erfindungsgemäß ist auch eine Verwendung eines erfindungsgemäßen Fixierungsimplantats in der Fertigungstechnik oder Medizintechnik.

Für das erfindungsgemäße Fixierungsimplantat wurde überraschend festgestellt, dass es zum Einen aufgrund des besonderen Designs des Drallaußengewindes einfach durch Aufbringung einer axialen Druckkraft selbstdrehend in einen vorgebohrten Knochen eingebracht werden kann, wobei diese Geometrie es erlaubt, kritische Torsionsbelastungen zu vermeiden. Der Einsatz eines Schraubendrehers mit den damit verbundenen Torsionsbelastungen ist nicht erforderlich. Zum Anderen erlaubt diese Geometrie den Einsatz von keramischen Materialen, die aufgrund der auftretenden Torsionsmomente bei herkömmlichen Interferenzschrauben für die vorgesehene medizinische Anwendung ausgeschlossen waren. Mit anderen Worten kann aufgrund der Form des Fixierungsimplantats keramisches Material eingesetzt werden, das den zur Einbringung erforderlichen Druckbelastungen ohne weiteres standhält. Die Fixierung des Fixierungsimplantats am Knochen kann erfindungsgemäß analog eines Keils über die Kontaktfläche des Drallaußengewindes des Fixierungsimplantats gewährleistet werden. Das erfindungsgemäße Fixierungsimplantat erlaubt somit die Erschließung einer neuen Materialklasse für solche Implantate, so dass die nachteiligen Eigenschaften beim Einsatz anderer Materialklassen, wie Polymer oder Metall, vermieden werden. Der operative Aufwand bei Einbringung des erfindungsgemäßen Fixierungsimplantats ist gering, das Bauteilversagen durch übermäßige Torsionslasten (Drehmomente) wird ebenfalls minimiert.

Im Grunde genommen gibt es zwei Arten von Spannungen, Druckspannungen und Zugspannungen. Das erfindungsgemäß eingesetzte keramische Material reagiert sehr empfindlich gegenüber Zugspannungen, wohingegen Druckspannungen deutlich stärker toleriert werden können.

Interferenzschrauben aus dem Stand der Technik werden bekanntlich mit einem Schraubendreher in den Bohrkanal eingebracht. Hierbei entstehen sehr hohe Zugspannungen, denen beispielsweise bioaktive Keramiken, wie Calciumphosphate oder Calciumsilikate, nicht standhalten können und brechen. Gemäß dem erfindungsgemäßen Fixierungsimplantat mit spezieller Schraubengeometrie wird kein Schraubendreher zum Einbringen mehr benötigt, sondern lediglich eine axiale Druckkraft, wie zum Beispiel einen Hammer. Daher entstehen beim erfindungsgemäßen Fixierungsimplantat kaum Zugspannungen, sondern vorrangig Druckspannungen, die vom keramischen Material toleriert werden können. Erfindungsgemäß wird daher zum Einbringen des Fixierungsimplantats lediglich eine axiale Druckkraft benötigt, um die Implantate einzubringen. Das "Eindrehen" des Fixierungsimplantats erfolgt designbedingt von allein.

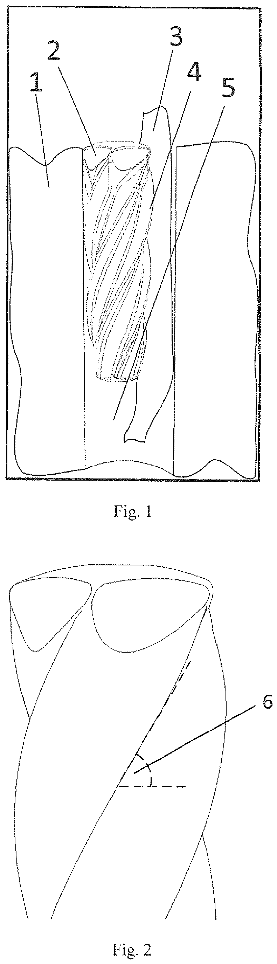

Weitere Merkmale und Vorteile des erfindungsgemäßen Fixierungsimplantats ergeben sich aus der folgenden detaillierten Beschreibung und der beigefügten Zeichnung, in der die Figur ein erfindungsgemäßes Fixierungsimplantat, eingesetzt zusammen mit einem Band in eine Knochenbohrung, in Schnittansicht zeigt. Fig. 2 veranschaulicht an einem beispielhaften Schraubenkörper, was unter einem Gewindesteigungswinkel zu verstehen ist.

Wie sich der in der Figur 1 gezeigten Schnittansicht entnehmen lässt, ist dort ein Fixierungsimplantat in Form einer Interferenzschraube 2 in einer Bohrung 5 innerhalb eines Knochenmaterials 1, zusammen mit einer Sehne oder einem Band 3, gezeigt. Die Interferenzschraube 2 weist ein Drallgewinde 4 auf, wobei sich das Drallgewinde 4 über den gesamten Implantatkörper der Interferenzschraube 2 erstreckt.

Zur Einführung der Interferenzschraube bohrt ein orthopädischer Chirurg in das Knochenmaterial 1 eine Bohrung 5 ein, was beispielsweise athroskopisch durchgeführt werden kann. Nachdem die Bohrung 5 hergestellt worden ist, wird ein Ende des Bandes 3 wie dargestellt positioniert. Die Interferenzschraube 2 wird dann durch Beaufschlagung einer axialen Druckbelastung in die Bohrung 5 selbstdrehend eingedreht, um das Band 3 in der Bohrung zu fixieren. Die Beauflagung der axialen Druckbelastung kann auf einfache Weise durch Beaufschlagung einer geeigneten Kraft auf die Stirnseite der Interferenzschraube 2 erfolgen.

Fig. 2 zeigt anschaulich anhand eines Schraubenkörpers, was unter einem Gewindesteigungswinkel zu verstehen ist Erfindungsgemäß ist es erforderlich, dass der Gewindesteigungswinkel größer als 30° ist. Ein solcher Steigungswinkel des Drallgewindes ermöglicht den Einsatz bzw. das Einbringen eines erfindungsgemäßen Fixierungsimplantats ohne ein aktives Eindrehen desselben.

## Patentansprüche

1. Fixierungsimplantat mit einem Implantatkörper, der im Wesentlichen aus keramischem Material aufgebaut ist und ein Drallaußengewinde aufweist, das mehr als einen Gewindesteg aufweist und wobei ein Gewindesteigungswinkel des Drallaußengewindes im Bereich von 30° bis 90° ist.

2. Fixierungsimplantat nach Anspruch 1, wobei das Fixierungsimplantat eine Interferenzschraube oder ein Anker, wie ein Fadenanker, ist.

3. Fixierungsimplantat nach Anspruch 1 oder 2, wobei das keramische Material ein biokompatibles keramisches Material, vorzugsweise ein Material auf Basis von Calciumphosphaten, Calciumsilikaten, Magnesiumsilikaten, Zinksilikaten und/oder Strontiumsilikaten, ist.

4. Fixierungsimplantat nach Anspruch 3, wobei das keramische Material auf Basis von Calciumphosphaten ausgewählt ist aus der Gruppe bestehend aus Hydroxylapatit, Tricalciumphosphat, Carbonatapatit, Fluorapatit und/oder Kombinationen derselben.

5. Fixierungsimplantat nach einem der vorangehenden Ansprüche, wobei das keramische Material ein Komposit-Material auf Basis von Polymer und keramischem Material oder Metall und keramischem Material ist.

6. Fixierungsimplantat nach einem der vorangehenden Ansprüche, wobei das Drallaußengewinde 2, 3, 4, 5 oder mehr im Wesentlichen parallel zueinander angeordnete Gewindestege aufweist.

7. Fixierungsimplantat nach einem der vorangehenden Ansprüche, wobei der Implantatkörper einen axialen zentrischen Kanal aufweist.

8. Fixierungsimplantat nach einem der vorangehenden Ansprüche, wobei der Implantatkörper im Wesentlichen konisch oder zylinderisch ausgebildet ist.

9. Fixierungsimplantat nach einem der vorangehenden Ansprüche, wobei das Drallaußengewinde sich im Wesentlichen über die gesamte Länge des Implantatkörpers erstreckt.

10. Verwendung eines Fixierungsimplantats nach einem der vorangehenden Ansprüche 1 bis 9 in der Fertigungstechnik oder Medizintechnik.

## Claims

1. Anchor implant comprising an implant body, which is made substantially of ceramic material and has an external helical thread, which has more than one thread web and wherein a thread pitch angle of the external helical thread ranges from 30° to 90°.

2. Anchor implant according to claim 1, wherein the anchor implant is an interference screw or an anchor, such as a suture anchor.

3. Anchor implant according to claim 1 or 2, wherein the ceramic material is a biocompatible ceramic material, preferably a material based on calcium phosphates, calcium silicates, magnesium silicates, zinc silicates and/or strontium silicates.

4. Anchor implant according to claim 3, wherein the ceramic material based on calcium phosphates is selected from the group consisting of hydroxyapatite, tricalcium phosphate, carbonate apatite, fluorapatite and/or combinations thereof.

5. Anchor implant according to one of the above claims, wherein the ceramic material is a composite material based on polymer and ceramic material or metal and ceramic material.

6. Anchor implant according to one of the above claims, wherein the external helical thread has 2, 3, 4, 5 or more thread webs arranged substantially parallel to one another.

7. Anchor implant according to one of the above claims, wherein the implant body has an axial central channel.

8. Anchor implant according to one of the above claims, wherein the implant body is configured to be substantially conical or cylindrical.

9. Anchor implant according to one of the above claims, wherein the external helical thread extends over substantially the entire length of the implant body.

10. Use of an anchor implant according to one of the above claims 1 to 9 in manufacturing technology or medical engineering.

## Revendications

1. Implant de fixation comprenant un corps d'implant, qui est composé essentiellement de matériau céramique et qui a un filetage hélicoïdal externe, ayant plus d'une nervure formant un filetage et dans lequel un angle de pas de filetage du filetage hélicoïdal externe se situe dans la plage de 30° to 90°.

2. Implant de fixation selon la revendication 1, dans lequel l'implant de fixation est une vis d'interférence ou un ancrage tel qu'un ancrage de suture.

3. Implant de fixation selon la revendication 1 ou 2, dans lequel le matériau céramique est un matériau céramique biocompatible, préférablement un matériau à base de phosphates de calcium, de silicates de calcium, de silicates de magnésium, de silicates de zinc et/ou de silicates de strontium.

4. Implant de fixation selon la revendication 3, dans lequel le matériau céramique à base de phosphates de calcium est sélectionné dans le groupe constitué d'hydroxyapatite, de phosphate de tricalcium, d'apatite carbonatée, de fluorapatite et/ou de combinaisons de ceux-ci.

5. Implant de fixation selon l'une quelconque des revendications précédentes, dans lequel le matériau céramique est un matériau composite à base de polymère et de matériau céramique ou de métal et de matériau céramique.

6. Implant de fixation selon l'une quelconque des revendications précédentes, dans lequel le filetage hélicoïdal externe a 2, 3, 4, 5 nervures formant un filetage ou davantage disposées de manière sensiblement parallèle les unes aux autres.

7. Implant de fixation selon l'une quelconque des revendications précédentes, dans lequel le corps de l'implant est doté d'un canal central axial.

8. Implant de fixation selon l'une quelconque des revendications précédentes, dans lequel le corps de l'implant est configuré pour être sensiblement de forme conique ou cylindrique.

9. Implant de fixation selon l'une quelconque des revendications précédentes, dans lequel le filetage hélicoïdal externe s'étend sensiblement sur toute la longueur du corps de l'implant.

10. Utilisation de l'implant de fixation selon l'une quelconque des revendications 1 à 9 dans la technologie de fabrication ou l'ingénierie médicale.
